# EUROPÄISCHE PATENTANMELDUNG

(11) **EP 0 825 154 A2**
(43) Veröffentlichungstag der Anmeldung: **25.02.1998**
(21) Anmeldenummer: 97114190.8
(22) Anmeldetag: 18.08.1997
(51) Int. Cl.: C02F 1/00, C02F 1/28, C02F 1/42, C02F 1/76, A61L 2/02

(54) **Vorrichtung zur Reinigung und Desinfektion von Wasser**

(30) Priorität: 19.08.1996 DE 19633352
(71) Anmelder: R-Amtech International, Inc., Bellevue, WA 98004 (US)
(72) Erfinder: Matyushin, Gennadiy Alexeevitch, 117 463 Moskau (RU); Kim, Zoya Kirillovna, 117 547 Moskau (RU)
(74) Vertreter: Hano, Christian, Dipl.-Ing.

(57) **Zusammenfassung**

Die Vorrichtung zur Reinigung und Desinfektion von Wasser weist ein röhrenförmiges Gehäuse (1) auf. Eine eintrittsseitige Filterstufe (10) umfaßt ein spulenförmiges Trägerelement (20) mit einem vorderen Flansch (22) und einem hinteren Flansch (18), die von einem Verbindungsabschnitt (17) verbunden werden. In dem Verbindungsabschnitt (17) ist ein koaxialer Kanal (21) ausgebildet, der in die vordere Oberfläche des vorderen Flansches (22) mündet und angrenzend an den hinteren Flansch (18) geschlossen ist. In der Außenfläche des Verbindungsabschnittes (17) sind mehrere Durchgangsöffnungen (24) vorgesehen, die in den Kanal (21) münden. Der Verbindungsabschnitt (17) ist von Filtermaterial (11) umgeben. Außerdem sind in der Wand des Gehäuses auf Höhe des Verbindungsabschnittes (17) mehrere Durchgangsbohrungen (23) vorgesehen.

## Beschreibung

Die Erfindung betrifft eine Vorrichtung zur Reinigung und Desinfektion von Wasser mit einem röhrenförmigen Gehäuse, in dem zwischen einem hinteren Eintrittsende und einem vorderen Austrittsende mehrere Stufen zur Reinigung bzw. Desinfektion vorgesehen sind.

Aus der US-A-4 298 475 ist eine gattungsgemäße Vorrichtung bekannt, in deren röhrenförmigem Gehäuse aus Kunststoff eine eintrittsseitige Stufe aus durchlässigem Material, eine weitere Stufe mit einer Polvhalogenidanionen-Austauschharzschicht, ein Zwischenfilter aus durchlässigem Polymer, ein Ausgangsfilter aus durchlässigem Polymer und ein Mundstück angeordnet sind. Aufgrund dieses Aufbaus wird Schmutzwasser ausreichend von Mikroorganismen desinfiziert und es werden mechanische Unreinheiten in einem gewissen Maße entfernt. Wenn mit der bekannten Vorrichtung beispielsweise Flußwasser mit einer anfänglichen Darmbakterienkontamination von 10⁵ Mikrobenzellen/Liter und mit einem Gehalt an mechanischen Verunreinigungen von 60 bis 100 mg/Liter fortlaufend aufgesogen wird, hat das das Austrittsende verlassende Wasser eine Kontamination an Darmbakterien von 10 bis 100 Mikrobenzellen/Liter und einen Gehalt an mechanischen Verunreinigungen von 40 bis 70 mg/Liter.

Diese Werte sind unzureichend. Außerdem ist die Menge an Wasser, die durch das Gehäuse strömen kann, gering, wenn das Wasser eine große Menge an Verunreinigungen organischen und sermenge, die mit einer solchen Vorrichtung aufgenommen werden kann, beträgt nicht mehr als 10 bis 15 l. Anschließend muß die bekannte Vorrichtung weggeworfen werden.

Aus der US-A-4 995 976 ist eine Vorrichtung mit einem röhrenförmigen Gehäuse aus Polycarbonat bekannt, bei dem die eintrittsseitige Stufe von einem Einlaßfilter aus durchlässigem Kunststoff mit einer Porengröße von 80 µm gebildet wird, der auf das eintrittsseitige Ende des Gehäuses aufgesteckt ist. An die eintrittsseitige Stufe schließt eine Reinigungsstufe an, die von einem Reinigungsharz gebildet wird und zur Abtötung von Bakterien, organischen Materialien und Organismen dient. Die nächste Stufe enthält Aktivkohle zur Absorption. Schließich ist eine Entkeimungsstufe vorgesehen. Die einzelnen Stufen sind durch durchlässige Distanzscheiben voneinander getrennt. Am vorderen und hinteren Ende der Aktivkohleschicht ist jeweils eine Schicht aus elastischem Schaum vorgesehen, die eine größere Porengröße besitzt als der Einlaßfilter oder die Distanzscheiben.

Mit dieser Vorrichtung läßt sich eine um eine Größenordnung verbesserte Reinigung und Desinfektion von Wasser erreichen als mit der aus der US-A-4 298 475 bekannten Vorrichtung. Die mit dieser Vorrichtung reinigbare Gesamtmenge an Wasser ist jedoch nicht größer.

Der Erfindung liegt die Aufgabe zugrunde, mit konstruktiv einfachen Mitteln eine Vorrichtung zur Reinigung und Desinfektion von Wasser zu schaffen, mit der es möglich ist, eine große Menge an Wasser ausreichend zu reinigen und desinfizieren.

Diese Aufgabe wird durch eine Vorrichtung mit den Merkmalen des Patentanspruchs 1 gelöst. Vorteilhafte Ausgestaltung mit der erfindungsgemäßen Vorrichtung sind Gegenstand der Patentansprüche 2 bis 12.

Die erfindungsgemäße Vorrichtung stellt eine ausreichende Desinfektion von Bakterien, Viren und Bakteriophagen unabhängig von der anfänglichen Verschmutzung des Wassers sicher. Darüberhinaus kann mit der erfindungsgemäßen Vorrichtung eine große Menge an Wasser gereinigt werden. Dies ist auf die Konstruktion der eintrittsseitigen Filterstufe mit dem spulenförmigen Trägerelement zurückzuführen, das von dem Filtermaterial umgeben ist. Aufgrund dieser Ausbildung hat die eintrittsseitige Filterstufe eine hohe Filterfläche zur Filterung mechanischer Verunreinigungen. Ein radialer Eintritt einer großen Wassermenge in die eintrittsseitige Filterstufe wird durch die Durchgangsbohrungen in der Wand des Gehäuses sichergestellt.

Ein Ausführungsbeispiel der Erfindung wird nachstehend anhand von Zeichnungen näher erläutert. Es zeigt:
- Fig. 1: einen Längsschnitt einer Vorrichtung zur Reinigung und Desinfektion von Wasser;
- Fig. 2: einen Längsschnitt eines perforierten Einsatzes;
- Fig. 3: einen Längsschnitt der eintrittsseitigen Stufe der Vorrichtung von Fig. 1 in vergrößertem Maßstab.

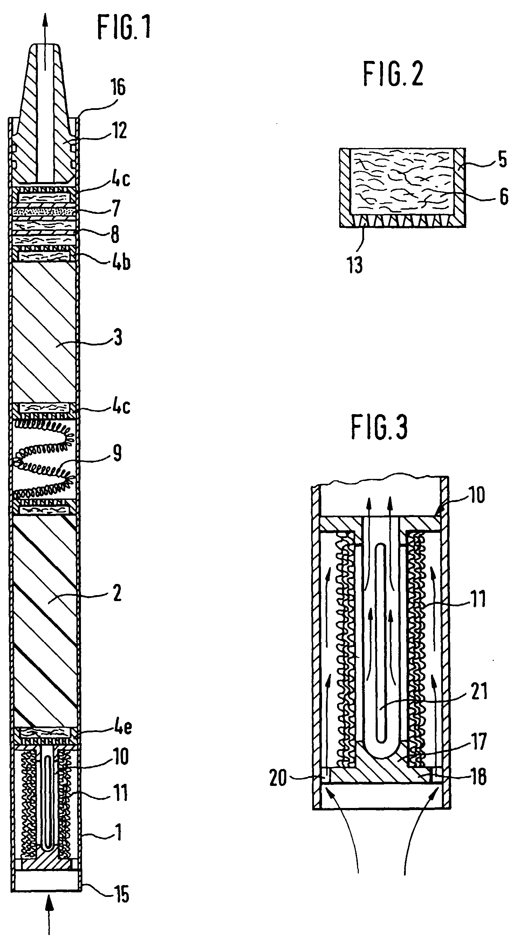

Die in Fig. 1 gezeigte Vorrichtung zur Reinigung und Desinfektion von Wasser weist ein zylindrisches röhrenförmiges Gehäuse 1 aus Polymer mit einem Eintrittsende 15 und einem Austrittsende 16 auf. In dem Gehäuse 1 sind mehrere Stufen zur Reinigung, Desinfektion und Entkeimung vorgesehen.

Am Eintrittsende 15 des Gehäuses 1 ist eine Filterstufe 10 angeordnet, die in Fig. 3 näher dargestellt ist. Die Filterstufe 10 umfaßt ein spulenförmiges Trägerelement 10 mit einem oberen Flansch 22 und einem unteren Flansch 18, deren Außendurchmesser im wesentlichen dem Innendurchmesser des Gehäuses 1 entsprechen. Die beiden Flansche 18, 22 sind durch einen länglichen zylindrischen Verbindungsabschnitt 17 miteinander verbunden, der koaxial zum Gehäuse 1 angeordnet ist. In dem Verbindungsabschnitt 17 ist ein sich koaxial erstreckender Kanal 21 ausgebildet, der in die vordere Oberfläche des vorderen Flanschs 22 mündet. Der Kanal 21 ist an seinem unteren Ende in der Nähe des unteren Flansches geschlossen. In der Seitenwand des Verbindungsabschnitts 17 sind mehrere (in dem gezeigten Beispiel vier) Längsschlitze 24 vorgesehen, die in gleichem Abstand um den Umfang des Verbindungsabschnitts 17 verteilt sind. Die Längsschlitze 24 münden in den Kanal 21 und in die Außenfläche des Verbindungsabschnittes 17.

Um den Verbindungsabschnitt 17 ist ein Filtermaterial 11 angeordnet, das von Aktivkohle enthaltenden Fasern gebildet wird. Für den Eintritt von Wasser sind in der Wand des Gehäuses 1 auf Höhe des Verbindungsabschnittes mehrere verteilte Durchgangsöffnungen 23 vorgesehen, die sicherstellen, daß eine ausreichende Menge an Wasser radial in die Filterstufe 10 eintreten kann.

Da der untere Flansch 18 abdichtend mit dem Gehäuse 1 verbunden ist und keine Durchgangsöffnungen aufweist, ist ein axialer Eintritt von Wasser in das Gehäuse 1 verhindert.

Die Durchgangsbohrungen 23 sind gleichmäßig um den Umfang des Gehäuses 1 verteilt. In dem gezeigten Beispiel sind jeweils drei Durchgangsöffnungen einem Längsschlitz 24 gegenüberliegend angeordnet. Größe, Anzahl und Anordnung der Durchgangsbohrungen kann jedoch auch anders gewählt werden, solange eine radiale Einströmung einer ausreichenden Menge an Wasser in die Filterstufe 10 gewährleistet ist.

Anschließend an die Filterstufe 10 ist ein zylindertopfförmiger Einsatz 4e angeordnet, der in Fig 2 vergrößert dargestellt ist. Der Einsatz 4e weist einen Boden 13 auf, der mit mehreren, gleichmäßig verteilten Durchgangsöffnungen 26 versehen ist. Der Raum zwischen der Zylinderwand 5 und dem Boden 13 des Einsatzes 4e ist mit Aktivkohle enthaltendem Fasermaterial 6 gefüllt. Der Außendurchmesser des Einsatzes 4e entspricht dem Innendurchmesser des Gehäuses 1. Der Einsatz 4e ist mit dem Boden 13 auf dem vorderen Flansch 22 der Filterstufe 10 aufgesetzt.

An die der Filterstufe 10 entgegengesetzten Seite des Einsatzes 4e grenzt eine Desinfektionsstufe 2 an, die beispielsweise aus desinfizierendem ionenaustauschendem Harz besteht, mit dem das Gehäuse 1 in diesem Bereich gefüllt ist. Die Desinfektionsstufe 2 wird an ihrem vorderen Ende durch einen weiteren Einsatz 4d begrenzt, der genauso ausgebildet ist, wie der Einsatz 4e, wobei der Boden des Einsatzes 4d von der Desinfektionsstufe 2 abgewandt ist.

Im Abstand zu dem Einsatz 4d ist ein weiterer gleich ausgebildeter Einsatz 4c so angeordnet, daß sein Boden dem Boden des Einsatzes 4d zugewandt ist. Der Einsatz 4c ist axial verschiebbar in dem Gehäuse 1 angeordnet, während der Einsatz 4d ortsfest angeordnet, d.h. unverschieblich ist. In dem Raum des Gehäuses 1 zwischen den Böden der Einsätze 4c und 4d ist eine spiralförmige Druckfeder 9 angeordnet, die aus gewundenem Aktivkohle enthaltendem Fasermaterial oder aus gewundenem Polymer besteht. Das Federelement 9 übt auf den Einsatz 4c eine nach vorne gerichtete Druckkraft aus.

Oberhalb des Einsatzes 4c ist eine Absorptionsstufe 3 vorgesehen, die Aktivkohle enthält, mit der das Gehäuse 1 in diesem Bereich gefüllt ist. Die Absorptionsstufe 3 wird an ihrem vorderen Ende durch einen weiteren unverschieblich angeordneten Einsatz 4b begrenzt, der wie der Einsatz 4e ausgebildet ist, wobei der Boden des Einsatzes 4b von der Absorptionsstufe 3 abgewandt ist.

Vor dem Einsatz 4b ist eine Entkeimungsstufe vorgesehen. Die Entkeimungsstufe weist drei Schichten 7 aus Entkeimungsmaterial auf, zwischen denen jeweils eine Zwischenschicht 8 aus Aktivkohle enthaltendem Fasermaterial vorgesehen ist. Angrenzend an die Entkeimungsstufe 7 ist ein weiterer Einsatz 4a angeordnet, der ebenfalls dem Einsatz 4e entspricht, wobei der Boden des Einsatzes 4a der Entkeimungsstufe abgewandt ist.

An den Einsatz 4a schließt ein Mundstück mit einem axial verlaufenden Durchgangskanal 27 an, das in das Austrittsende 16 des Gehäuses 1 eingesteckt ist.

Die Höhe der Filterstufe 10 beträgt ungefähr 10 bis 12 % der Höhe des Gehäuses 1. Das Verhältnis zwischen der Höhe der Desinfektionsstufe und der Absorptionsstufe liegt zwischen 5 : 4 und 2 : 1. Als Material für die Desinfektionsstufe kann ein Polyhalogenidanionen-Austauschharz oder ein anderes Harz mit analogen bakterientötenden Eigenschaften verwendet werden. Das Polyhalogenid kann Iod, Brom oder eine Kombination davon sein. Es können auch fünf oder mehr Zwischenschichten aus Aktivkohle enthaltendem Fasermaterial verwendet werden.

Zur Aufnahme von Wasser wird die erfindunsgemäße Vorrichtung mit ihrem Eintrittsende 15 in eine verschmutzte Wasserquelle gehalten. An dem Mundstück 12 wird ein Unterdruck erzeugt. Das Wasser tritt durch die Durchgangsbohrungen 23 in der Wand des Gehäuses 1 in den Raum zwischen dem Trägerelement 20 und der Wand des Gehäuses 1 ein und strömt im wesntlichen radial durch das Filtermaterial 11 aus Aktivkohle enthaltendem Fasermaterial und die Längsschlitze 24 in den Kanal 21 des Verbindungsabschnitts 17. Durch das Filtermaterial 11 werden mechanische Verunreinigungen aus dem Wasser entfernt.

Anschließend durchströmt das Wasser die Desinfektionsstufe 2, den Einsatz 4d, den Einsatz 4c und die Absorptionsstufe 3 aus Aktivkohle, in der im Wasser gelöste Substanzen absorbiert werden. Hier wird der Geschmack des gereinigten Wassers verbessert und außerdem der Anteil an verbleibendem Iod oder ähnlichem in dem Wasser verringert. Durch die Druckfeder 9 wird über den Einsatz 4c ein Druck auf die Absorptionsstufe 3 ausgeübt, wodurch verhindert wird, daß sich in der Absorptionsstufe 3 Kanäle bilden, durch die das Wasser ohne ausreichende Absorption strömen könnte.

Nach der Absorptionsstufe 3 strömt das Wasser durch die Schichten 7 aus Entkeimungsmaterial und die Zwischenschichten 8 aus Aktivkohle enthaltendem Material, wobei das Wasser durch die Schichten 8 normalisiert wird. Außerdem wird eine zusätzliche Filtrierung und Absorption von in dem Wasser aufgelösten Substanzen durch die Zwischenschichten 8 ausgeführt. Der Reinigungsgrad erhöht sich mit der Anzahl der Zwischenschichten aus Aktivkohlefasermaterial.

Schließlich tritt das Wasser in das Mundstück 12 ein, von wo aus es verwendet werden kann.

Die erfindungsgemäße Vorrichtung wurde hinsichtlich ihrer Durchgangsleistung, ihres Reinigungsgrades im Hinblick auf toxische organische und mechanische Verunreinigungen, ihrer Desinfektionswirkung, dem Wasserdurchsatz durch die Vorrichtung und dem Gehalt an Desinfektionsmaterial in dem gereinigten Wasser getestet.

Mit Durchsatzleistung ist die Strömungsmenge gemeint, die die Vorrichtung durchströmt bis der Durchsatz durch die Vorrichtung dreimal geringer ist als der anfängliche Durchsatz. Die Desinfektionswirkung wird bezüglich des Gehalts an Darmbakterien in dem gereinigten Wasser bestimmt. Nach der Norm der Russischen Föderation GOST 2874-82, ist Wasser, dessen Gehalt an Darmbakterien größer als drei Mikrobenzellen/Liter ist, zum Trinken ungeeignet. Der Reinigungsgrad des Wassers bezüglich mechanischer Verunreinigung ist das Verhältnis zwischen den Verunreinigungen in dem gereinigten Wasser und den Verunreinigungen in dem eintretenden Wasser. Der Strömungsdurchsatz durch die Vorrichtung ist die Wassermenge (in ml), die in einer Minute die Vorrichtung durchströmt, wenn zwischen dem Eintrittsende und dem Austrittsende ein Druckunterschied von 0,2 atm besteht. Wenn als Desinfektionsmaterial ein Polyiodanionen-Austauschharz verwendet wird, wird der Gehalt an Desinfektionsmaterial im gereinigten Wasser anhand des Gehalts an Iod im gereinigten Wasser bestimmt. Wenn der Anteil von Iod größer als 4 mg/Liter ist, hat das Wasser einen schlechten Geruch und ist zum Trinken ungeeignet. Der Reinigungsgrad des Wassers bezüglich toxischer organischer Verunreinigungen wird durch das prozentuelle Verhältnis der Konzentration dieser Verunreinigungen in dem Wasser vor und nach dem Durchgang durch die Vorrichtung bestimmt.

Bei einem Test der erfindungsgemäßen Vorrichtung erzeugte eine Pumpe einen Unterdruck von 0,2 atm. Es wurden 0,5 l Flußwasser pro Tag durch die Vorrichtung gesogen, um die erreichbare Strömungsmenge der Vorrichtung zu ermitteln.

Das Flußwasser hatte eine anfängliche Kontamination mit Darmbakterien von 10⁵ Mikrobenzellen/Liter und einen Verschmutzungsgrad an mechanischen Verunreinigungen von 60 mg/Liter.

Das Gehäuse hatte eine Länge von 200 mm und einen Innendurchmesser von 10 mm. Die Höhe der Filterstufe 10 betrug 25 mm bzw. 12,5 % der Länge des Gehäuses 1. Das Verhältnis der Höhe zwischen der Desinfektionsschicht 2 aus Polyiodharz SIA-1 (TU 64-2-381-87) und der Absorptionsschicht 3 mit aktivierter Ligninkohle betrug 3:2. Als Entkeimungsmittel wurde Silber enthaltendes kationenaustauschendes Material mit einem Silberionengehalt von 8,5 mg/Liter mit einer Körnigkeit von 0,5 bis 1,2 mm verwendet. Die Höhe der Entkeimungsstufe betrug 10 mm bzw. 5 % der Länge des Gehäuses.

Mit dieser Vorrichtung wurde eine Durchgangsleistung von 25 l erreicht. Der Gehalt an Darmbakterien in dem gereinigten Wasser betrug weniger als 3 Mikrobenzellen/Liter. Es wurde ein Reinigungsgrad für mechanische Verunreinigungen von 10 % erreicht. Der Durchsatz an Wasser durch die Vorrichtung betrug 125 ml/Minute. Der Iodgehalt des aus der Vorrichtung austretenden Wassers betrug 2,5 mg/Liter.

## Patentansprüche

1. Vorrichtung zur Reinigung und Desinfizierung von Wasser, mit einem röhrenförmigen Gehäuse (1), in dem zwischen einem hinteren Eintrittsende (15) und einem vorderen Austrittsende (16) mehrere Stufen zur Reingung bzw. Desinfizierung vorgesehen sind,
dadurch gekennzeichnet, daß die eintrittsseitige Filterstufe (10) ein spulenförmiges Trägerelement (20) umfaßt, das einen hinteren (18) und einen vorderen Flansch (22) aufweist, deren Außendurchmesser im wesentlichen dem Innendurchmesser des Gehäuses (1) entspricht und die mittels eines länglichen, zylindrischen Verbindungsabschnitts (17) miteinander verbunden sind, der koaxial in dem Gehäuse (1) angeordnet ist,
wobei
- in dem Verbindungsabschnitt (17) ein koaxialer Kanal (21) ausgebildet ist, der in die vordere Oberfläche des vorderen Flansches (22) mündet und angrenzend an den hinteren Flansch (18) geschlossen ist,
- in der Außenfläche des Verbindungsabschnittes (17) mehrere Durchgangsöffnungen (24) vorgesehen sind, die in den Kanal (21) münden,
- der Verbindungsabschnitt (17) von Filtermaterial (11) umgeben ist, und
- in der Wand des Gehäuses (1) auf Höhe des Verbindungsabschnitts (17) mehrere Durchgangsbohrungen (23) vorgesehen sind.

2. Vorrichtung nach Anspruch 1, dadurch gekennzeichnet, daß das Filtermaterial (11) von Aktivkohle enthaltenden Fasern gebildet wird, die um den Verbindungsabschnitt (17) gewickelt sind.

3. Vorrichtung nach Anspruch 2, dadurch gekennzeichnet, daß das Filtermaterial (11) aus Aktivkohle enthaltenden Fasern eine Durchlässigkeit von 0,4 cm³/g aufweist.

4. Vorrichtung nach einem der vorhergehenden Ansprüche, dadurch gekennzeichnet, daß
zwischen der eintrittsseitigen Stufe (10) und dem Austrittsende (16) eine Desinfektionsstufe (2) und eine Aktivkohle enthaltende Absorptionsstufe (3) vorgesehen sind, an deren vorderen Ende und hinteren Ende jeweils ein perforierter zylinderischer Einsatz (4b, 4c, 4d, 4e) angeordnet ist, dessen Aussendurchmesser im wesentlichen dem Innendurchmesser des Gehäuses (1) entspricht, wobei der vordere (4d) und der hintere (4e) Einsatz der Desinfektionsstufe (2) sowie der vordere Einsatz (4b) der Absorptionsstufe (3) ortsfest und der hintere Einsatz (4e) der Absorptionsstufe (3) axial verschieblich angeordnet sind und zwischen dem vorderen Einsatz (4d) der Desinfektionsstufe (2) und dem hinteren Einsatz (4e) der Absorptionsstufe (3) ein Federelement (9) vorgesehen ist, das auf den hinteren Einsatz der Absorptionsstufe eine Druckkraft in Richtung der Absorptionsstufe ausübt.

5. Vorrichtung nach Anspruch 4, dadurch gekennzeichnet, daß das Federelement (9) aus gewundenem Polymer oder gewundenem, Aktivkohle enthaltendem Material besteht.

6. Vorrichtung nach einem der Ansprüche 4 oder 5, dadurch gekennzeichnet, daß das Volumen des Raums zwischen dem vorderen Einsatz (4d) der Desinfektionsstufe (2) und dem hinteren Einsatz (4c) der Absorptionsstufe (3) 20 bis 100% des Volumens der Desinfektionsstufe (2) beträgt.

7. Vorrichtung nach einem der Ansprüche 4 bis 6, dadurch gekennzeichnet, daß das Verhältnis zwischen dem Volumen der Desinfekktionsstufe (2) und dem Volumen der Absorptionsstufe (3) 5:4 bis 2:1 beträgt.

8. Vorrichtung nach einem der Ansprüche 4 bis 7, dadurch gekennzeichnet, daß die Aktivkohle der Absorptionsstufe (3) von aktiver Ligninkohle mit einer Körnigkeit von 0,5 bis 1,5 mm gebildet wird.

9. Vorrichtung nach einem der Ansprüche 4 bis 8, dadurch gekennzeichnet, daß zwischen dem vorderen Einsatz (4b) der Absorptionsstufe (3) und dem Austrittsende (16) eine Entkeimungsstufe (7) vorgesehen ist, die Entkeimungsmaterial enthält, in dem wenigstens eine Zwischenschicht (8) aus Aktivkohle enthaltenden Fasern angeordnet ist.

10. Vorrichtung nach Anspruch 9, dadurch gekennzeichnet, daß zwischen der Entkeimungsstufe (7) und dem Austrittsende (16) ein perforierter zylinderischer Einsatz (4a) angeordnet ist, dessen Aussendurchmesser im wesentlichen dem Innendurchmesser des Gehäuses (1) entspricht.

11. Vorrichtung nach einem der Ansprüche 4 bis 10, dadurch gekennzeichnet, daß die Einsätze (4a, 4b, 4c, 4d, 4e) topfförmig sind und einen durchlässigen Boden (13) aufweisen, der von der zugeordneten Stufe abgewandt ist, wobei zwischen dem Boden und der entsprechenden Stufe Aktivkohle enthaltende Fasern (6) angeordnet sind.

12. Vorrichtung nach einem der vorhergehenden Ansprüche, dadurch gekennzeichnet, daß an dem Austrittsende (16) ein Mundstück (12) vorgesehen ist.
